Europäisches Patentamt

European Patent Office

Office européen des brevets

(19)

(11) Publication number: **0 116 236**
**B1**

(12) **EUROPEAN PATENT SPECIFICATION**

(45) Date of publication of patent specification: **04.02.87**

(51) Int. Cl.⁴: **A 61 F 2/24**

(21) Application number: **83308022.9**

(22) Date of filing: **29.12.83**

(54) Heart valve replacements.

(30) Priority: **11.01.83 GB 8300636**

(43) Date of publication of application:
**22.08.84 Bulletin 84/34**

(45) Publication of the grant of the patent:
**04.02.87 Bulletin 87/06**

(84) Designated Contracting States:
**AT BE CH DE FR GB IT LI LU NL SE**

(56) References cited:
**FR-A-2 391 708**
**US-A-4 084 268**
**US-A-4 106 129**
**US-A-4 192 020**

(73) Proprietor: **THE UNIVERSITY OF SHEFFIELD**
**Western Bank**
**Sheffield, S10 2TN (GB)**

(72) Inventor: **Black, Martin Morris**
**26 Broad Elms Lane**
**Sheffield S11 9QR (GB)**
Inventor: **Drury, Philip John**
**43 Winchester Crescent**
**Sheffield S11 4ED (GB)**
Inventor: **Tindale, Wendy Belinda**
**6 Knab Road**
**Sheffield S7 2ET (GB)**

(74) Representative: **Hulse, Thomas Arnold et al**
**Hulse & Co. Cavendish Buildings West Street**
**Sheffield S1 1ZZ (GB)**

EP 0 116 236 B1

Courier Press, Leamington Spa, England.

## Description

This invention relates to heart valve replacements, of the type comprising a two-part frame formed by a first frame part and a second frame part, each of which frame parts is substantially ring-shaped, and at least one leaflet having a margin passing through a nip formed between the two frame parts.

Such a heart valve replacement is disclosed in US—A—4 192 020 in which a plurality of leaflets are sealed to one of the frame parts.

The object of the invention is to provide a heart valve replacement of the type initially described in which the at least one leaflet is secured to one of the frame parts by means of stitching, but avoiding any possibility of flexure or hinging of the leaflet being coincident with or interrupted by. a line of sutures or stitches, thus preventing any possibility of tearing between stitch holes.

According to the present invention, a heart valve replacement of the type initially described is characterised in that each of said first and second frame parts has a cloth-covering, the margin of the leaflet which passes through the nip between the two frame parts being stitched to the cloth-covering of the first frame part, and said first and second frame parts being secured together by stitching between the cloth coverings on the outside of both parts. Thus the flexure or hingeing line of the leaflet will be along the nip between the two cloth-covered frame parts, which line is intermediate the main body of the leaflet and the stitching both of its margin to the cloth-covering of the first frame part and of the cloth-covering of both frame parts to each other, completely eliminating any possibility of tearing between stitch holes.

The frame parts are preferably formed of Delrin (registered Trade Mark) and covered with Dacron (registered Trade Mark) cloth.

The first frame part, to the cloth-covering of which the margin of the leaflet is initially stitched, is preferably wire-like in character, so that the margin of the leaflet can be wrapped round it to up to half a turn or more whilst still conforming to the profile of that frame part or part of its profile, which may be flat or convoluted depending on the detailed nature of the heart value replacement, while the second frame part may be a portion of a cylinder with a convoluted profile to match a convoluted profile of the first frame part, and the cloth-covering of the second frame part may be united with a cloth-covering of a sewing ring (which ring is preferably formed of P.T.F.E.) before stitching the cloth-coverings of the two frame parts together.

An embodiment of the invention will now be described by way of example only, with reference to the accompanying drawings, in which:—

Figure 1 is an enlarged perspective view of the first part (hereinafter called the "female" part) of a two-part frame for a heart valve replacement in accordance with the invention;

Figure 2 is a perspective view, to the same scale as Figure 1, of the second part (hereinafter called the "male" part) of the frame;

Figure 3 corresponds to Figure 1 but shows the female part covered with cloth;

Figure 4 is an underneath plan of the female part as shown in Figure 3;

Figure 5 corresponds to Figure 3 but shows two leaflets stitched to the cloth covering of the female part;

Figure 6 is a further enlarged fragmentary section taken on the line VI—VI of Figure 5;

Figure 7 corresponds to Figure 2 but shows the male part covered with cloth, which cloth covering is united with a cloth covering of a sewing ring;

Figure 8 is a fragmentary section, to the same scale as Figure 6, taken on the line VIII—VIII of Figure 7;

Figure 9 is a perspective view, to the same scale as the preceding perspective views, showing the completed heart valve replacement (together with its sewing ring);

Figure 10 is an underneath plan of the heart valve replacement as shown in Figure 9; and

Figure 11 is a fragmentary section, to the same scale as Figures 6 and 8, taken on the line XI—XI of Figure 9.

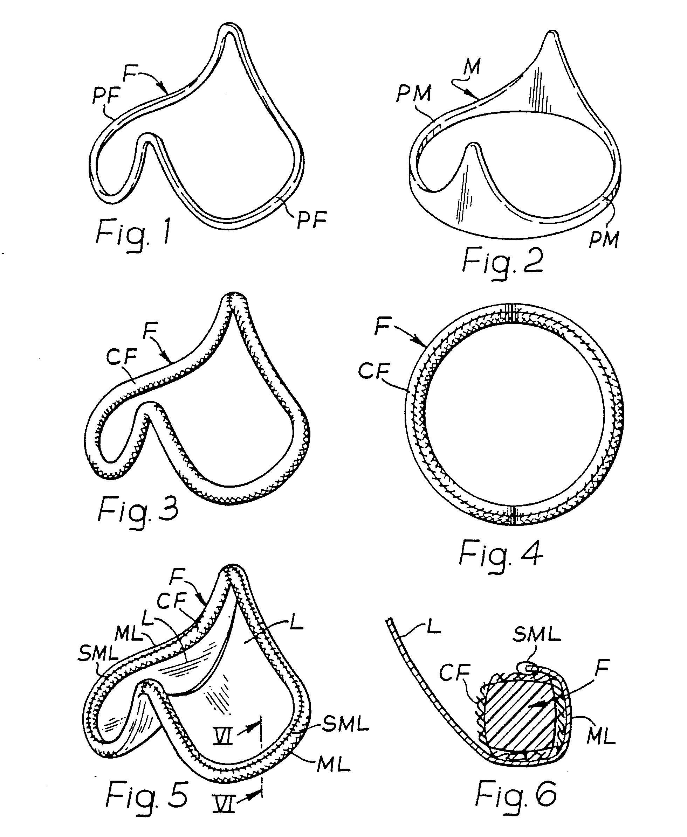

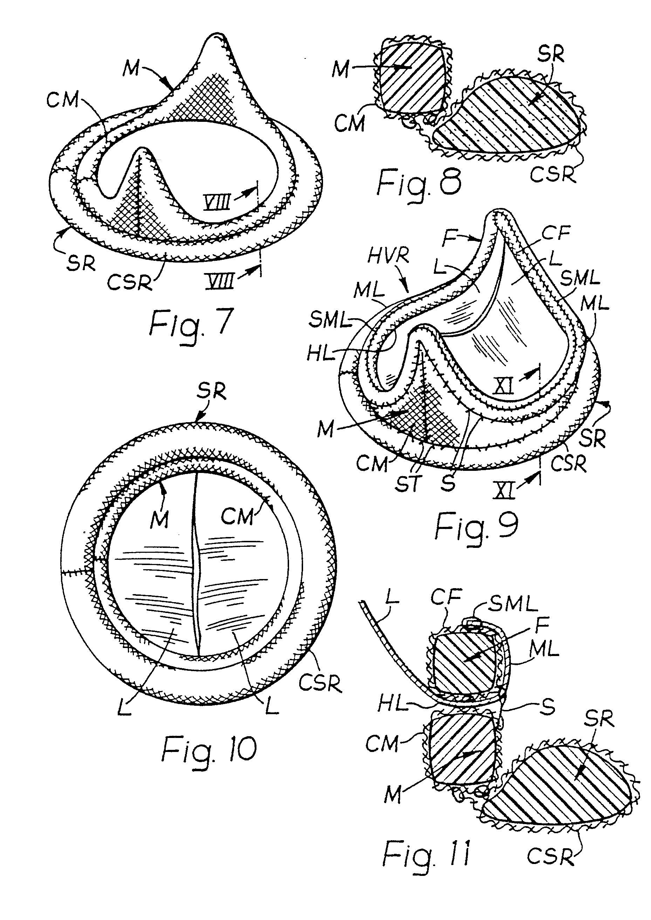

The heart valve replacement HVR (shown complete with a sewing ring SR in Figures 9 and 10) comprises a two-part frame consisting of a female part F (Figure 1) and a male part M (Figure 2), with each part generally ring-shaped and covered with cloth CF (Figure 3) and CM (Figure 7) respectively, and a pair of leaflets L each having a margin ML passing through a nip formed between the two parts of the frame (see Figure 11) and stitched to the cloth-covering CF of the female part F, the frame parts F, M being secured together by stitching S between the cloth-coverings CF, CM on the outside of both parts.

Thus the flexure or hingeing line HL of each leaflet L will be along the nip between the cloth-covered frame parts, which line is intermediate the main body of the leaflet and the stitching SML of its margin ML to the covering CF of the female frame part F and the stitching S of the cloth-coverings CF,CM of both frame parts F,M to each other, completely eliminating any possibility of tearing between stitch holes.

The frame parts F, M are formed of Delrin (registered Trade Mark) and covered with Dacron (registered Trade Mark) cloth CF,CM.

The female frame part F (to the cloth-covering of which the margin ML of each leaflet L is initially stitched — see also Figures 5 and 6) is wire-like in character, so that the margin ML of each leaflet L can be wrapped round it to half a turn or more whilst still conforming to part PF the profile of the female frame part, which is convoluted (because the heart valve replacement HVR is a bicuspid mitral valve replacement) and the male frame part M is a portion of a cylinder with a convoluted profile PM to match the profile of the female part.

The cloth-covering CM of the male frame part M is united with a cloth-covering CSR of the sewing ring SR (which is formed of P.T.F.E.) and — as indicated by Figures 7 and 8 — before stitching the cloth-coverings CF, CM of the two frame parts F,M together (as shown in Figures 9 to 11). Thus the cloth-covering CM resembles a pair of trousers with the cloth-covering CSR resembling its waistband.

Finally, the cloth-covering CSR of the sewing ring SR is preferably stitched to the cloth-covering CM of the male frame part M as shown at ST in Figure 9, to maintain the general position of the sewing ring relative to the frame parts F,M as shown by Figure 11 without inhibiting the ability of the sewing ring to conform to the respective aperture in a heart.

**Claims**

1. A heart valve replacement comprising a two-part frame formed by a first frame part (F) and a second frame part (M) each of which frame parts is substantially ring-shaped, and at least one leaflet (L) having a margin (ML) passing through a nip formed between the two frame parts, characterised in that each of said first and second frame parts (F, M) has a cloth-covering (CF,CM), the margin (ML) of the leaflet (L) which passes through the nip between the two frame parts being stitched to the cloth covering (CF) of the first frame part (F), and said first and second frame parts (F, M) being secured together by stitching (S) between the cloth-coverings (CF,CM) on the outside of both parts.

2. A heart valve replacement as in Claim 1, characterised in that the first frame part (F) is wire-like in character.

3. A heart valve replacement as in Claim 2, characterised in that the wire-like first frame part (F) is convoluted, and in that the second frame part (M) is a portion of a cylinder with a convoluted profile (PM) to match the convoluted profile (PF) of the first frame part (F).

4. A heart valve replacement as in as in any one of Claims 1 to 3, characterised in that the cloth-covering (CM) of the second frame part (M) is united with a cloth-covering (CSR) of a sewing ring (SR) before stitching the cloth-coverings (CF,CM) of the two frame parts (F,M) together.

**Patentansprüche**

1. Herzklappenersatz mit einem zweiteiligen Rahmen aus einem ersten Rahmenteil (F) und einem zweiten Rahmenteil (M), deren jeder im wesentlichen ringförmig ist, und mit wenigstens einem Klappenblatt (L) mit einem Rand (ML), der durch einen zwischen den beiden Rahmenteilen gebildeten Spalt hindurchgeführt ist, dadurch gekennzeichnet, daß jeder der beiden Rahmenteile (F, M) einen Stoffbezug (CF, CM) aufweist, der durch den Spalt zwischen den zwei Rahmenteilen hindurchgeführte Rand (ML) des Klappenblattes (L) an den Stoffbezug (CF) des ersten Rahmenteiles (F) angenäht ist und das erste sowie zweite Rahmenteil (F, M) durch auf deren Außenseite angeordnete Nahtstiche (S) zwischen den Stoffbezügen (CF, CM) der Rahmenteile miteinander verbunden sind.

2. Herzklappenersatz nach Anspruch 1, dadurch gekennzeichnet, daß das erste Rahmenteil (F) eine drahtähnliche Gestalt aufweist.

3. Herzklappenersatz nach Anspruch 2, dadurch gekennzeichnet, daß das drahtähnliche erste Rahmenteil (F) gewunden ist und das zweite Rahmenteil (M) Teil eines Zylinders mit einer zur gewundenen Gestalt (PF) des ersten Rahmenteiles (F) passenden gewundenen Kontur (PM) ist.

4. Herzklappenersatz nach einem der Ansprüche 1 bis 3, dadurch gekennzeichnet, daß der Stoffbezug (CM) des zweiten Rahmenteiles (M) vor dem gegenseitigen Vernähen der Stoffbezüge (CF, CM) der beiden Rahmenteile (F, M) mit einem Stoffbezug (CSR) eines Nähringes (SR) vereinigt worden ist.

**Revendications**

1. Valvule cardiaque de remplacement comprenant une structure de support en deux parties formée par une première partie (F) et une seconde partie (M), chacune de ces parties de la structure de support étant sensiblement de forme annulaire, et au moins un feuillet (L) comportant une partie marginale (ML) passant par une zone de pincement formée entre les deux parties de la structure de support, caractérisée en ce que chacune desdites première et seconde partie (F, M) de la structure de support comprend un élément de recouvrement en tissu (CF, CM), la partie marginale (ML) du feuillet (L) qui passe par la zone de pincement entre les deux parties de la structure de support étant consue à l'élément de recouvrement en tissu (CF) de la première partie (F) de la structure de support, et lesdites première et seconde parties de la structure de support (F, M) étant rendues solidaires par une couture (S) entre les éléments de recouvrement en tissu (CF, CM) sur le côté extérieur des deux parties.

2. Valvule cardiaque de remplacement selon la revendication 1, caractérisée en ce que la première partie (F) de la structure de support est du type à fil métallique.

3. Valvule cardiaque de remplacement selon la revendication 2, caractérisée en ce que la première partie (F) de la structure de support qui est du type à fil métallique est convolutée, et en ce que la seconde partie (M) de la structure de support est une partie d'un cylindre à profil convoluté (PM) pour se coformer au profil convoluté (PF) de la première partie (F) de la structure de support.

4. Valvule cardiaque de remplacement selon l'une quelconque des revendications 1 à 3, caractérisée en ce que l'élément de recouvrement en tissu (CM) de la seconde partie (M) de la structure de support est unie à un élément de recouvrement en tissu (CSR) d'un anneau de couture (SR) avant la couture l'un à l'autre des éléments de recouvrement en tissu (CF, CM) des deux parties (F, M) de la structure de support.

Fig. 1

Fig. 2

Fig. 3

Fig. 4

Fig. 5

Fig. 6

1

Fig. 7

Fig. 8

Fig. 9

Fig. 10

Fig. 11